# EUROPEAN PATENT APPLICATION

(11) **EP 4 613 187 A1**
(43) Date of publication of application: **10.09.2025**
(21) Application number: 24162031.9
(22) Date of filing: 07.03.2024
(51) Int. Cl.: A61B 5/024, A61B 5/107, A61B 5/11, A61B 5/113, G01L 1/24

(54) **MONITORING PATIENT STATE IN CONNECTION WITH MEDICAL PROCEDURES**

(71) Applicant: Koninklijke Philips N.V., 5656 AG Eindhoven (NL)
(72) Inventor: DENISSEN, Adrianus Johannes Maria, Eindhoven (NL); ALBERTS, Eveline, Eindhoven (NL); KRUISKAMP, Marinus Johan, 5656AG Eindhoven (NL); STEMKENS, Bjorn, Eindhoven (NL); VERSLUIS, Maarten Jan, Eindhoven (NL); HEUVELINK, Annerieke, Eindhoven (NL); HUIJBERS, Willem, Eindhoven (NL); MATTERS, Marco, Eindhoven (NL); WESTERINK, Joanne Henriëtte Desirée Monique, Eindhoven (NL)
(74) Representative: Philips Intellectual Property & Standards

(57) **Abstract**

There is provided a method for monitoring patient state in connection with a medical procedure. The method comprises: obtaining pressure signals from at least one optical waveguide pressure sensor array arranged to interact with the patient; and processing the pressure signals to monitor patient state. A corresponding patient state monitoring system is also provided.

## Description

### FIELD OF THE INVENTION

The invention relates to methods and systems for monitoring patient state in connection with medical procedures.

### BACKGROUND OF THE INVENTION

Pressure sensing systems which rely on the use of optical fibers as the sensor, such as that described in WO 2017/144675 A1, have been used for monitoring movements of persons during sleep. However, the full potential of such systems is yet to be realized.

### SUMMARY OF THE INVENTION

To better address one or more of these concerns, there is provided, in a first aspect of the invention, a method as defined by the following non-exhaustive list of non-limiting examples. Any one or more of the features of these examples may be combined with any one or more features of any other example, embodiment, or aspect described herein.

A method for monitoring patient state in connection with a medical procedure, the method comprising:
obtaining pressure signals from at least one optical waveguide pressure sensor array arranged to interact with the patient; and
processing the pressure signals to monitor patient state.

The medical procedure may comprise a diagnostic imaging procedure. The diagnostic imaging procedure may comprise a magnetic resonance imaging procedure, a computed tomography imaging procedure, an X-ray imaging procedure, or an ultrasound procedure. The medical procedure may comprise an interventional procedure. The interventional procedure may comprise catheterization or surgery. The medical procedure may comprise a therapeutic procedure. The therapeutic procedure may comprise radiotherapy, physical therapy, or substance administration. The medical procedure may comprise any combination of procedures such as those identified herein.

The method may be performed to monitor patient state during the medical procedure, prior to the medical procedure, and/or following the medical procedure.

The method may further comprise using the monitored patient state for planning and/or preparation of the medical procedure, for example, for protocol setting in relation to an image acquisition process. Protocol setting may comprise planning elements (boundary setting, margin setting), device acquisition parameters and/or protocol settings (e.g. MR sequences, X-ray dose for CT or diagnostic X-ray, etc.), and/or path determination. Examples of parameters that may be involved in protocol setting include for example a delay in starting acquiring or the duration of acquisition after reaching a certain state and the topic of informed reconstruction.

The method may further comprise using the monitored patient state for patient positioning. In the case that the location of the one or more parts of the patient is determined based on the pressure signals, the determined location may be used in patient positioning. Using the determined location of the one or more parts of the patient in patient positioning may comprise changing a pose of the one or more parts of the patient based on the determined location. Using the determined location of the one or more parts of the patient in patient positioning may comprise changing the location of the one or more parts of the patient based on the determined location.

The method may further comprise using the pressure signals obtained from at least one optical waveguide pressure sensor array for placement of equipment in connection with the medical procedure. The equipment may comprise one or more sensors, coils, protective devices, or entertainment devices.

The method may further comprise obtaining further pressure signals from the at least one optical waveguide pressure sensor array during a planning phase prior to the medical procedure, and verifying that the patient position remains unchanged during the planning phase. The method may further comprise obtaining further pressure signals from the at least one optical waveguide pressure sensor array during a planning phase prior to the medical procedure to perform thereby a position verification scan. The method may further comprise verifying that the patient position remains unchanged with reference to one or more predetermined acceptability criteria defining acceptable deviations, based for example on one or more acceptable tolerances, similarity metrics, and/or rules. The method may further comprise, in response to detecting an unacceptable change in patient position during the planning phase, stopping the planning phase. The method may further comprise, in response to detecting an unacceptable change in patient position during the planning phase, updating the medical procedure, or an image acquisition plan thereof. If the surrogate is quantitative, the pressure signals may be used to perform a simple 'virtual couch shift' to eliminate the need for replanning. For example, when quantitatively a position shift from planning to acquisition is measured, instead of signaling this to the operator that should then (manually) replan, this shift is used to automatically update the plan (e.g., shift the field of view).

The pressure sensor array may be used for the detection of the location or motion of one or more body parts of the patient. In particular, processing the pressure signals may comprise detecting location or motion of one or more body parts of the patient. Body parts of the patient may comprise a wrist, forearm, humerus, elbow, collarbone, foot, ankle, tibia-fibula, femur, chest, lung, rib, hip, pelvis, abdomen, spine, skull, upper airway, jaw, mandible, sinus, head, neck, knee, shoulder, leg, and/or a hand of the patient or internal organs such as the heart, lungs, bowels, etc.

Detecting patient motion may comprise detecting changes in patient position occurring between completion of patient positioning and completion of the medical procedure. The method may comprise, at a first timepoint, receiving a user-input signal indicating the completion of the patient positioning, and storing the pressure signals obtained at the first timepoint from the at least one optical waveguide pressure sensor array as a reference pressure footprint for use in detecting changes in patient position. The method may further comprise storing the reference pressure footprint for future use in one or more follow-up procedures. The user-input signal indicating the completion of the patient positioning may be received from a user interface component forming part of, or connected to, the at least one optical waveguide pressure sensor array. Detecting changes in patient position may comprise continuously or repeatedly comparing the pressure signals currently obtained from the at least one optical waveguide pressure sensor array with one or more reference pressure footprints. The method may further comprise signaling a detected change in patient position to one or more users. The one or more users may comprise a technologist and/or the patient. Signaling the detected change may comprise presenting a traffic light visualization based on the magnitude of the detected change relative to one or more threshold magnitudes. The one or more threshold magnitudes may vary according to pose, the method further comprising obtaining an indication of the current pose of the patient and selecting the one or more threshold magnitudes based thereon. The method may further comprise, in response to detecting a change in patient position, generating one or more output signals for output to one or more users, the output signals comprising information to assist the patient in returning to a correct patient position. The method may further comprise generating one or more output signals for output to one or more users to indicate that the medical procedure is complete. The method may further comprise storing data related to detected changes in the patient's record. In the case that the medical procedure comprises an image acquisition process, the method may further comprise using data related to detected changes as input for one or more subsequent image processing steps. The one or more image processing steps comprise one or more image correction or stitching operations. Detecting changes in patient position may comprise using data related to pose assistance material used during patient positioning. The medical imaging may comprise diagnostic X-ray imaging, MR, CT, PET, SPECT or combinations thereof.

Detecting patient motion may comprise estimating a magnitude of the detected patient motion. Detecting patient motion may comprise estimating an origin of the detected patient motion.

The method may further comprise, in response to detecting patient motion, outputting one or more output signals for triggering remedial action. In the case that the medical procedure comprises an image acquisition process, the remedial action may comprise changing, guiding, or adapting the image acquisition process in response to detecting the patient motion. The output signals may instruct an imaging system performing the image acquisition process to initiate one or more adaptations to the image acquisition process, for example live protocol adaptations. The adaptations to the image acquisition process may comprise one or more corrections, changing acquisition parameters or triggering. The remedial action may comprise repeating at least a portion of the image acquisition process, wherein the output signals instruct an imaging system performing the image acquisition process to repeat at least the portion of the image acquisition process. The remedial action may comprise notifying or alerting one or more users of the detected patient motion, wherein the output signals instruct one or more user interface devices to inform the one or more users about the detected patient motion. The one or more users may comprise the patient. The image acquisition process may comprise quantitative imaging, the method further comprising using the detected patient motion to improve signal quantification. For example, the detected pressure signal changes may be translated into movement action of the patient to restore the original position after excessive movement. Additionally or alternatively, the patient may be asked to move incrementally until the original position is restored, as indicated for example by traffic light 'green' or OK signal, because most of the time the patient does not know which movement they made.

Detecting patient motion may comprise detecting patient respiratory activity. Detecting patient motion may comprise detecting patient cardiac activity. Detecting patient motion may comprise detecting patient cardiorespiratory activity. Detecting the patient motion may comprise detecting patient respiratory activity and patient cardiac activity simultaneously. In the case that the medical procedure comprises a tomographic scan such as an MR or CT scan, the method may further comprise using the detected patient cardiac activity for cardio timing of the tomographic scan.

Processing the pressure signals to monitor patient state may comprise determining one or more patient body measurements. The body measurements may comprise one or more of: patient body weight; patient body height; geometric measurement. The method may further comprise detecting a change in one or more of the body measurements. Detecting the change may comprise detecting an interfractional change and/or an intrafractional change. Any one or more such patient body measurements may be used in procedure planning and/or set-up.

Processing the pressure signals to monitor patient state may comprise determining a location of one or more parts of the patient. Determining the location of one or more parts of the patient may comprise locating one or more patient organs. In the case that the medical procedure comprises an image acquisition process, the method may further comprise outputting the location of the one or more parts of the patient to an imaging system performing the image acquisition process. The method may further comprise recentering the imaging system based on the location of the one or more parts of the patient. The method may comprise performing image registration and/or scaling based on the location of the one or more parts of the patient. Image registration (e.g. of MR and CT images or multiple MR-images acquired during subsequent MR scans) may be employed in imaging for treatment planning. When the images are planned differently (e.g. between the MR and CT scan) there may be variation in the field of view, requiring scaling to obtain images of the same size that can be registered to each other.

Processing the pressure signals to monitor patient state may comprise determining a level of filling of one or more patient organs. The one or more patient organs may comprise a bladder. The one or more patient organs may comprise a rectum. The one or more patient organs may comprise a bowel. Detecting the change in the one or more body measurements may comprise detecting a change in the level of filling of the one or more patient organs and alerting a user and/or adapting the medical procedure accordingly.

Processing the pressure signals to monitor patient state may comprise comparing a first patient position determined at a first timepoint with a second patient position determined at a second timepoint to identify a deviation in patient position. The first patient position may comprise a previous patient position used during a previous medical procedure, which may comprise an image acquisition process. The first patient position may serve as a reference patient position for subsequent monitoring. The second patient position may comprise a current patient position. The method may further comprise using the deviation in patient position to align the current patient position with the reference patient position. Processing the pressure signals to monitor patient state comprises detecting patient respiratory activity, the method further comprising comparing respiratory activity at the first timepoint with respiratory activity at the second timepoint to identify a respiratory synchronization error. The respiratory activity at the first timepoint serves as a reference respiratory activity for subsequent monitoring. The method may further comprise using the respiratory synchronization error to align current respiratory activity with the reference respiratory activity. The current respiratory activity may comprise one or more of a current respiration type, depth, and rate, wherein aligning the current respiratory activity with the reference respiratory activity comprises aligning one or more of the current respiration type, depth, and rate with a reference respiration type, depth, and rate, respectively.

Detecting patient respiratory activity may comprise detecting patient respiratory activity at multiple locations. The multiple locations may comprise locations in the chest and/or abdomen of the patient. The method may further comprise using the detected respiratory activity to estimate motion of one or more patient organs. Detecting patient respiratory activity may comprise using calibration data in the detection. The method may further comprise using the detected patient respiratory activity to characterize patient breathing. Respiratory activity is periodic and thereby detectable after a number of cycles. Breathing motion that is characterized using the pressure signals may be used to guide image acquisition or treatment delivery.

Detecting patient motion may comprise detecting patient bowel motion. In the case that the medical procedure comprises an image acquisition process, the method may further comprise, in response to detecting patient bowel motion, recentering an imaging system performing the image acquisition process to exclude at least part of a region in which patient bowel motion is detected. The method may comprise, in response to detecting patient bowel motion, instructing the imaging system performing the image acquisition process to stop or suspend the image acquisition process. Detecting patient bowel motion comprises detection motion of an air pocket in the patient's bowel, wherein the image acquisition process is stopped or suspended in response to the air pocketing entering a radiation field. The method may further comprise detecting patient bowel activity before the image acquisition process and estimating based thereon a movement impact on one or more patient organs.

In any of these ways, the monitored patient state may be used for guiding the medical procedure, for example by moving an image center based on detected location or motion.

Processing the pressure signals to monitor patient state may comprise recognizing one or more gestures performed by the patient to provide user input. The one or more gestures may comprise one or more tapping gestures. The one or more gestures may comprise one or more swipe gestures. The method may further comprise calibrating the at least one optical waveguide pressure sensor array in least a region of the patient's hand for recognition of the one or more gestures. The method may further comprise raising an alarm in response to recognizing an alarm gesture performed by the patient. The method may further comprise using the user input in relation to the performance of a task during task-based fMRI. The method may further comprise using the user input in connection with real-time neurofeedback.

Detecting patient motion may comprise detecting patient motion in two or more dimensions. The method may further comprise using the detected patient motion in two or more dimensions to detect changes in patient respiration. The method may further comprise using the detected patient motion in two or more dimensions to detect changes in patient respiration from chest-to-belly breathing to back breathing or vice versa.

In the case that the medical procedure comprises an image acquisition process in combination with a treatment process, the method may further comprise, in response to detecting patient motion, controlling an imaging system performing the image acquisition process to suspend or stop irradiation. The method may further comprise, in response to detecting patient motion, updating a treatment plan. For still images of organs without breathing motion, such as the brain or foot, the MRI reconstruction may use several shots of the organ at the same position in 3D space. The pressure signals can be used to inform the MRI acquisition system which shots contain excessive motion and which therefore need to be retaken without necessarily repeating the whole scan. The method may further comprise, in response to detecting patient motion, comparing a post-motion patient position with a pre-motion patient position, and initiating remedial action when the post-motion patient position differs from the pre-motion patient position by more than a predetermined amount. Initiating remedial action may comprise issuing a warning. Initiating remedial action may comprise outputting corrective guidance to a user. In the case that the medical procedure comprises a treatment process, the pressure signals may be used (in addition to, or instead of, image-guiding) to inform users and/or treatment systems when to start and stop irradiation (i.e. gated treatment).

In the case that the medical procedure comprises an image acquisition process, processing the pressure signals to monitor patient state may comprise detecting contact or proximity during the image acquisition process between one or more parts of the patient and one or more dangerous objects. The one or more parts of the patient may comprise one or more regions of skin and the one or more dangerous objects comprise one or more further regions of the patient's skin. The one or more dangerous objects may comprise one or more radiofrequency transmission coils. The one or more dangerous objects may comprise one or more cables. Processing the pressure signals to monitor patient state may comprise detecting skin-to-skin contact or proximity. The method may further comprise, in response to detecting the contact or proximity, issuing a warning.

The patient state monitored on the basis of the obtained pressure signals may be used in image reconstruction. More particularly, the monitored patient state may be used to determine which images are to be used in image reconstruction and/or to determine weights to be attached to images for use in image. reconstruction. For example, the monitored patient state may be used to perform multidimensional sorting of 4D-MRI data, in the case that the medical procedure comprises an MRI scan. The MRI system may capture a subset of image information of a 3D space around a region of interest, e.g. an organ. In each phase of the breathing, the organ of interest can be in a different position. For good image quality, multiple shots of the organ in exactly the same position may be needed. The pressure signals may be used to determine which shots of each breathing phase are similar in 3D space so as to sort or group corresponding shots to form one still image per breathing phase (in a process commonly referred to as binning). In this way, a 4D image for example of a beating heart can be visualized in 3D over time. The method of the first aspect may be at least partially computer implemented.

According to a second aspect, there is provided a computing system configured to perform the method of the first second aspect. The computing system may comprise, or be comprised in, the patient state monitoring system described herein.

According to a third aspect, there is provided a computer program comprising instructions which, when executed by a computing system, enable or cause the computing system to perform the method of the first aspect.

According to a fourth aspect, there is provided a computer-readable medium comprising instructions which, when executed by a computing system, enable or cause the computing system to perform the method of the first aspect. The computer-readable medium may be transitory or non-transitory, volatile or non-volatile.

By monitoring patient state during medical imaging using at least one optical waveguide pressure sensor array arranged to interact with the patient during an image acquisition process, in the manner described herein, image quality can be improved and the efficiency of radiotherapy of moving organs increased. In particular, image quality can be improved for example by determining and accounting for motion artifacts caused by the patient's movement, respiration, and/or heartbeat.

The pressure sensing system as described herein can measure ballistic heartbeat and small weight changes during breathing in multiple dimensions. These many-dimensional signals enable stroboscopic imaging or radiation to find the organ under inspection/radiation always in the same position.

For scanning exams that are relatively long for the patient, somewhere ranging between roughly 2 and 40 minutes, it is difficult for the patient to keep lying perfectly still. The pressure signals as described herein can be used to estimate the movement and the imaging system may be corrected during the scan, or individual scans with motion artifacts may be discarded.

By detecting breathing and heart rate using the pressure sensing system as described herein, no sensors need be attached to the patient, which provides less montage work for the technologist and more comfort for the patient. Moreover, the body shape contour is no longer disturbed by the mounting of a respiration belt and image quality is no longer disturbed by ECG stickers or wires. Additionally, no extra scan sequences are needed to estimate the position on the diaphragm (the so-called navigator).

As used herein, the term "patient" refers to the human or animal subject of a medical imaging examination and does not necessarily denote a person of ill health. That is, the term "patient" may be applied to the subject regardless of the state of health of the subject or the environment in which the medical imaging is performed.

As used herein, the term "patient state" may comprise one or more of: a position of the patient or part thereof; a motion of the patient or part thereof; a condition of the patient or part thereof. The patient state may comprise for example a medical state of the patient, as indicated for example by one or more vital signs, or a state or condition of one or more biological processes of the patient, for example as indicated by one or more biomarkers. In particular examples described herein, "patient state" may be used to refer to the sedation level of the patient, or the state of the cardiorespiratory activity of the patient, the state of internal organs of the patient, for example the filling level of one or more internal organs, e.g. bowel, bladder, and so on. As an example of patient condition, the patient state may comprise a patient geometry, that is, one or more body measurements or geometrical measurements relating to the patient. Such measurements may relate to one or more physiological parameters and/or anthropometric parameters characterizing the patient, e.g. dimensional descriptors of body size and shape, e.g. height, weight, body mass index (or BMI), waist-to-hip ratio, and so on.

As used herein, the term "patient position", as one example of patient state, may refer in particular to a location and/or posture of the patient or part thereof. The location and/or posture may be defined relative to one or more references, for example a previous location or posture, some part of the imaging apparatus, such as the patient support, or another part of the patient. The patient position may alternatively be referred to herein as a "pose".

Accordingly, the term "patient motion" may be understood in terms of a deviation in patient position between two or more different timepoints. "Detecting patient motion" as described herein may thus comprise obtaining pressure signals corresponding to two or more different timepoints, and detecting patient motion based on one or more differences between the pressure signals corresponding respectively to the two or more different timepoints. To that end, the pressure signals may therefore be obtained continuously, periodically, or repetitively. In this way, detecting patient motion may comprise tracking patient position. The obtained pressure signals may thus comprise both spatial and temporal data, i.e. spatiotemporal data. The pressure signals may thus be used to obtain a static or dynamic occupancy pressure distribution whereby patient state including patient position and/or patient motion may be detected. Patient motion as described herein may be interfractional, that is, variations in patient position occurring from one treatment fraction to the next, possibly because of variability in patient positioning or volumetric changes in a tumor, for example. Additionally or alternatively patient motion may by interfractional, that is, variations in patient position occurring during a single treatment fraction, causing geometric alterations from moment to moment. Patient motion may comprise intentional and/or unintentional patient movement, that is, conscious and/or unconscious patient motion. Intentional or conscious patient motion may comprise deliberate position changes or user gestures for communication purposes, for example. Unintentional or unconscious patient motion may comprise for example internal motion, usually organ motion, resulting from physiological processes such as cardiorespiratory activity (respiration, heartbeat), organ filling, gastrointestinal activity such as peristalsis, or urinary activity. Organ motion may be defined as the mobility of organs relative to the bony anatomy, for example. Patient motion may thus involve micromovements and/or macromovements.

In contrast to "patient position", the term "patient positioning" as used herein refers to the act of putting the patient into a desired patient position on the patient support. Patient positioning may alternatively be referred to herein as "posing".

By "patient support" is meant that part of the imaging apparatus which supports the patient, for example an examination table.

As used herein, the term "monitoring" refers to the acquisition of data defining the patient state at at least one point in time, preferably at several timepoints, for example in a continuous, periodic or repetitive manner.

As used herein, the term "medical imaging" refers to the process of imaging at least part of the interior or exterior of a body for clinical analysis and medical intervention, and comprises techniques such as radiography, magnetic resonance imaging, ultrasound, computed tomography, and so on.

As used herein, the term "quantitative imaging" refers to the extraction of quantifiable features from medical images for the assessment of normal or of the severity, degree of change, or status of a disease, injury, or chronic condition relative to normal.

As used herein, the term "remedial action" refers to one or more corrective actions and/or preventive actions and/or actions to discard unwanted information. For example, taking remedial action may comprise stopping, suspending, updating, or repeating at least part of an imaging plan or treatment plan, and/or interacting with one or more users, for example by issuing a warning, providing a report, or providing user output, and so on.

The term "circuitry", as used herein, may comprise, for example, singly or in any combination, hardwired circuitry, programmable circuitry such as computer processors comprising one or more individual instruction processing cores, state machine circuitry, and/or firmware that stores instructions executed by programmable circuitry.

The term "obtaining", as used herein, may comprise, for example, receiving from another system, device, or process; receiving via an interaction with a user; loading or retrieving from storage or memory; measuring or capturing using sensors or other data acquisition devices.

The term "determining", as used herein, encompasses a wide variety of actions, and may comprise, for example, calculating, computing, processing, deriving, investigating, looking up (e.g., looking up in a table, a database or another data structure), ascertaining, and the like. Also, "determining" may comprise receiving (e.g., receiving information), accessing (e.g., accessing data in a memory), and the like. Also, "determining" may comprise resolving, selecting, choosing, establishing and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

These and other aspects of the invention will be apparent from and elucidated with reference to the embodiments described hereinafter.

### BRIEF DESCRIPTION OF THE DRAWINGS

A detailed description will now be given, by way of example only, with reference to the accompanying drawings, in which:
Fig. 1 is a schematic diagram of a pressure sensing system;
Fig. 2 illustrates pressure sensing systems such as that shown in Fig. 1 being used to sense pressure exerted by a patient;
Figs. 3A-B illustrate pose assistance material which may be used to facilitate detection of patient motion using the pressure sensing system of Fig. 1;
Fig. 4 illustrates rotation of the head during medical imaging;
Figs. 5A and 5B illustrate aspects of an optical waveguide pressure sensor array;
Fig. 6 illustrates cardiorespiratory activity detected by the pressure sensing system of Fig. 1;
Fig. 7 illustrates unsafe skin-to-skin contact during an MRI scan; and
Fig. 8 illustrates a computing system that can be used in accordance with the systems and methods disclosed herein.

### DETAILED DESCRIPTION OF EMBODIMENTS

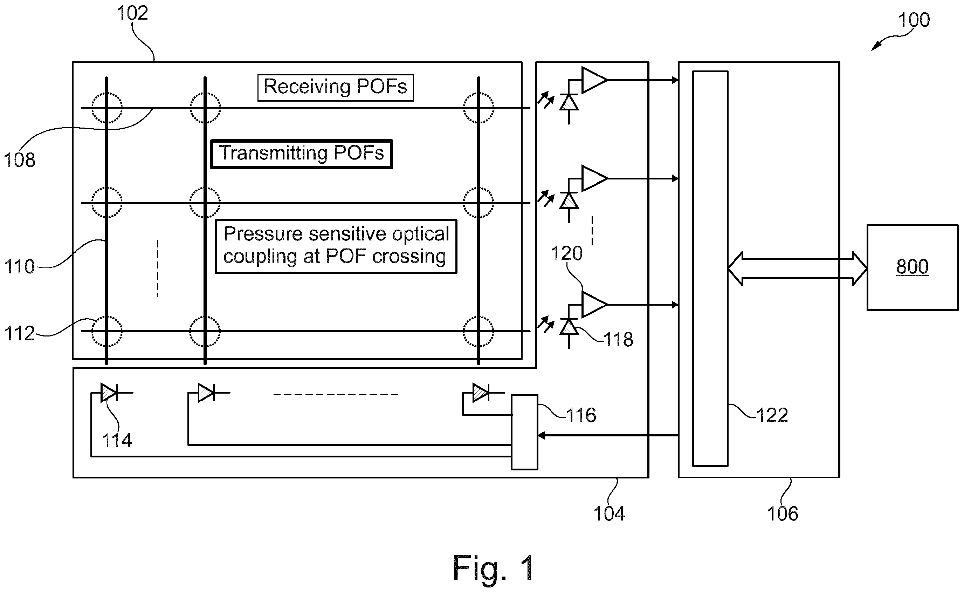

Fig. 1 is a schematic diagram of a pressure sensing system 100. The pressure sensing system 100 comprises an optical waveguide pressure sensor array 102, optoelectronic circuitry 104, and data acquisition and control circuitry 106.

The optical waveguide pressure sensor array 102 comprises a plurality of row optical waveguides, such as that indicated at 108, and a plurality of column optical waveguides, such as that indicated at 110. The optical waveguides 108, 110 preferably comprise polymer or plastic optical fibers (POF). The row optical waveguides 108 and the column optical waveguides 110 are deformable and are arranged in a planar array or matrix to define a sensor at each crosspoint, such as that indicated at 112. Each crosspoint 112 thus includes one of the row optical waveguides 108 in contact with one of the column optical waveguides 110 at the intersection of those two waveguides. Each crosspoint 112 may further comprise a light coupling structure (not shown) configured to enhance waveguide bending when pressure is applied to the crosspoint 112. The light coupling structure may comprise at least one layer of mechanical light scattering material disposed in contact with at least one of the row or column optical waveguides 108, 110. The light coupling structures may be applied on both sides of the row and column optical waveguides 108, 110, and may have a layer of light scattering material either on one side, or on both sides. In one non-limiting example, each row optical waveguide 108 includes a waveguide core surrounded by a waveguide cladding, and each column optical waveguide 110 includes a waveguide core surrounded by a waveguide cladding, wherein the waveguide claddings of both the row optical waveguides 108 and the column optical waveguides 110 are arranged for light transmissive contact at one or more of the crosspoints 112. The cladding is sufficiently thin that a non-negligible amount of light may be coupled from the core of one waveguide to the crossing waveguide. At an increased ratio between cross-sectional diameter of the core to the cladding, the light has a higher tendency to couple into the crossing waveguide. As such, the ratio may be at least 50:1 but more preferably is higher.

The optoelectronic circuitry 104 comprises a plurality of LEDs 114, with each LED 114 being connected to a respective one of the column optical waveguides 110, which thereby serve as transmitting waveguides. A selector 116 is configured to enable selective activation of the LEDs 114 by the data acquisition and control circuitry 106. The optoelectronic circuitry 104 further comprises a plurality of photodiodes 118, each coupled to a respective row optical waveguide 108 to receive light therefrom. The row optical waveguides 108 thus serve as receiving optical waveguides. It will, however, be understood that the column optical waveguides 110 may alternatively serve as the receiving optical waveguides and that the row optical waveguides 108 may serve as the transmitting optical waveguides. The optoelectronic circuitry 104 further comprises a plurality of amplifiers 120 for amplifying the signals produced by respective photodiodes 118. Since the optical coupling between transmitting and receiving optical waveguides is small, high-sensitivity optical receivers are needed. The amplifiers 120 may therefore comprise transimpedance amplifiers (TIAs) with high gain and a high input impedance. The amplified signals are output to the data acquisition and control circuitry 106.

The data acquisition and control circuitry 106 controls the LEDs 114 and processes the signals obtained from the photodiodes 118. To that end, the data acquisition and control circuitry 106 comprises interface circuitry 122 for interfacing with the optoelectronic circuitry 104. The interface circuitry 122 may comprise one or more analog-to-digital converts for converting the analog signals output by the amplifiers 120 to digital signals. Preferably, for improving the SNR of sensors, the LEDs are designed and/or controlled so that the full range of the analog-to-digital converters can be used. The digital pressure signals may then be output to a patient state monitoring system 800 for further processing as described hereinbelow. The patient state monitoring system 800 and/or the data acquisition and control circuitry 106 may be configured to perform one or more preprocessing operations, such as resizing, orienting, scaling, filtering, segmenting, reducing noise, and so on, for rendering the pressure signals more suitable for the further processing operations.

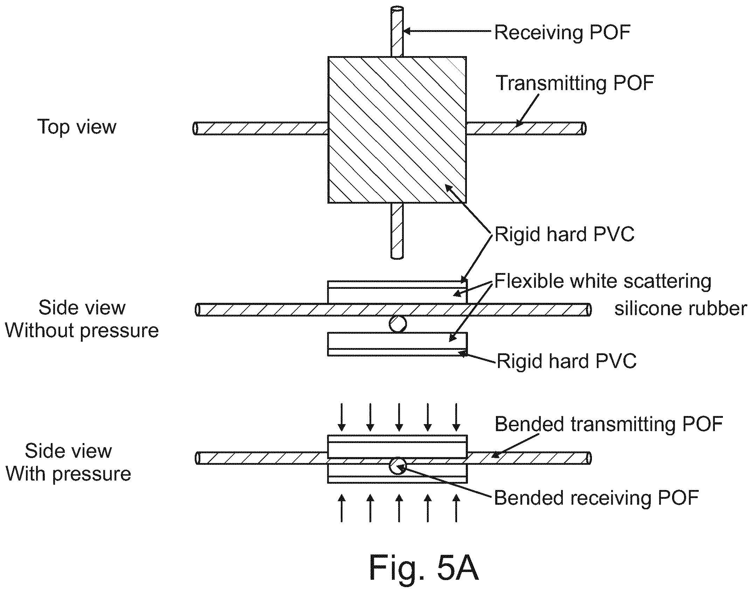

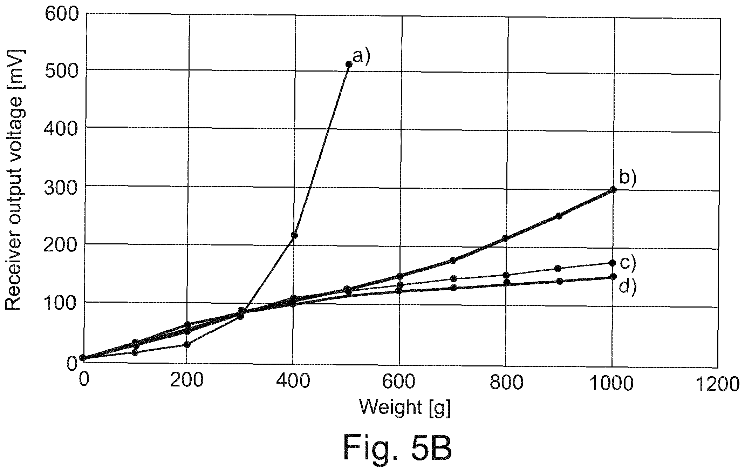

Figs. 5A and 5B illustrate the structure of the optical waveguide pressure sensor array 102 in detail.

In use, the optical waveguide pressure sensor array 102 is configured to sense pressure, when light is provided to the row optical waveguides 108, by measuring the light coupled at each crosspoint 112 to its column optical waveguide 110 (or vice versa). The amount of coupled light is dependent on the pressure applied to the crosspoint 112, which thereby acts as a pressure sensor. Moreover, as only a small amount of light is coupled out of the transmitting optical waveguide 110 when pressure is exerted on the respective crosspoint 112, the light power which remains in the transmitting optical waveguide 110 and which arrives at the next crosspoint 112 is hardly reduced. Thus, the sensitivity of a crosspoint 112 is hardly affected by the pressure on other crosspoints 112, i.e. the location-dependence of the performance of a crosspoint 112 is negligible. There is therefore provided a two-dimensional optical waveguide pressure sensor array 102 having high sensitivity. With such a pressure sensor array 102, the two-dimensional spatial distribution of pressure of objects and/or persons on a certain structure can be measured.

In one example of a pressure sensing operation performed by the pressure sensing system 100, a crosspoint scanning method is implemented. The data acquisition and control module 106 controls the selector 116 to activate only one LED 114 at a time and scans the crosspoints 112 column-by-column. Reading the amplified signals output by the photodiodes 118 line-by-line enables the data acquisition and control circuitry 106 to perform 2D pressure sensing. This solution is readily scalable because N photodiodes and M LEDs can sense pressure at N x M crosspoints when the columns are scanned one-by-one.

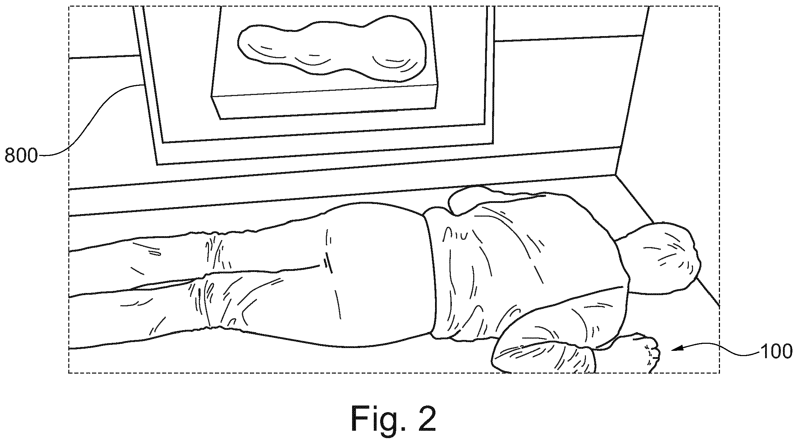

During the image acquisition process, the pressure sensor array 102 is arranged to interact with the patient such that the pressure signals may represent the patient state. That is, the sensor array 102 is positioned for direct or indirect contact with at least part of the patient, i.e., with at least one body part of the patient, preferably that part which is relevant for the subsequent processing as described below. By "indirect" is meant that there may be intervening objects between the patient and the sensor array 102. For example, Fig. 2A depicts the pressure sensed by the pressure sensing system 100 when positioned underneath a mattrass with the patient lying on the mattrass. The pressure sensing system 100 is not prevented by the indirect contact from outputting useful information by virtue of its sensitivity. In Fig. 2A, the patient state monitoring system 800 is shown as obtaining the pressure signals from the pressure sensing system 100 and displaying a representation of the 2D sensed pressure on a display. As described further below, the pressure sensing system 100 and/or the patient state monitoring system 800 may comprise one or more user interface components for interfacing with the user, such as a technologist and/or a patient.

According to the present disclosure, the pressure sensing system 100 is used for monitoring patient state during medical imaging to improve picture quality, speed and comfort. By installing the pressure sensing system 100 on or in the table of a CT, MR, Xray or PET scanner for radiotherapy, the state of the patient (such as body position and respiration) can be monitored and also harmonized across different imaging modalities. Since the pressure sensing system 100 is thin (e.g. around 1mm thickness), flexible, and robust, it can be attached to the MR, CT, X-ray, PET or RT table, for example by adhesive, without interfering with the imaging or radiation system. Aspects of the patient state such as the beating heart, respiration and body movement of the patient thereby become detectable. The present disclosure envisages numerous different use cases for the pressure sensing system 100 in the field of medical imaging, as will now be described.

### Use case #1: detecting patient motion

The patient can perform subtidal motion without noticing during image acquisition, which can lower the image quality. For example, movement of the legs during a head scan can disturb the homogeneity of the magnetic field and thereby lower the image quality. Even MR-generated peripheral nerve stimulation (PNS, see http://mriquestions.com/nerve-stimulation.html) could be detected, which is generally not observed by the technologist or noticeable by the patient. The pressure sensing system 100 can detect these movement phenomena, whose size and origin may then be estimated by the patient state monitoring system 800, which may further signal the MRI machine to skip and repeat some scanning sequences or raise an alarm to the technologist.

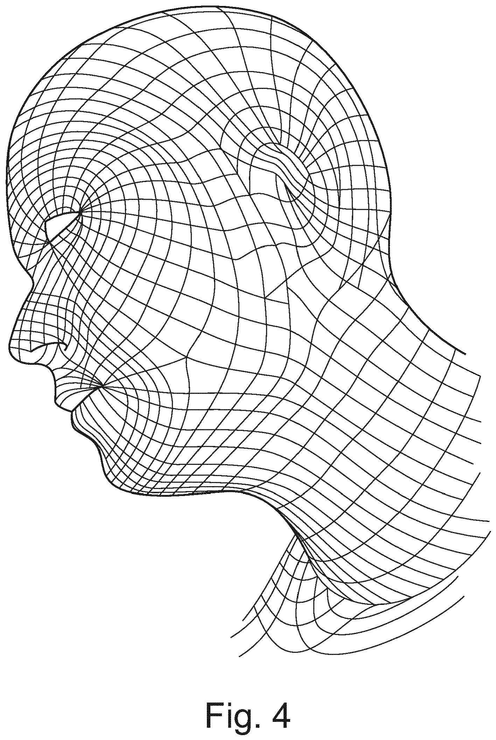

For scanning sequences that are relatively long for the patient, for example up to 45 minutes, it is very hard for the patient to lay perfectly still. Using a pressure sensing system such as that described with reference to Fig. 1 positioned underneath the pillow of the MR head coil, as depicted in Fig. 2B, it is possible to detect small head movements and head rotations. Fig. 4 shows that the effect of a small rotation of the head is mainly visible in the outermost region of the head: voxels farther from the center move more through 3D space. In one non-limiting example, the pressure sensing system is able detect translations with an accuracy of 0.1mm and rotations with an accuracy of 0.1 degree. A 4D head calibration sequence during which the patient for instance slowly shakes their head to indicate "yes" and "no" for 30 seconds is enough to relate head movements to the signals output by the pressure sensing system 100. During the subsequent (long) scan, the pressure signals can be used to correct the MR acquisition process in real-time so that less post processing is needed for the best image quality. Movements which are too large can be detected in real-time with the pressure sensing system 100 and the MR scanner can be instructed to redo the corresponding dynamics. The same movement estimations can be used to inform the patient (for example via a mirror) about their head position relative to the starting position. In this way the patient immediately understands what 'keep your head still' means. When a rotation or translation is detected which is deemed too large, this visual information is helpful to encourage the patient to return their head to the starting position without redoing the whole scan sequence.

The pressure sensing system 100 may be specially configured to monitor movement of the patient's head-and-neck, knee, shoulder, legs or hands, for example by its being sized and shaped to embed in the special coils. The pressure sensing system 100 is sufficiently sensitive to be able to detect swallowing, coughing, talking, singing, sighing, and yawning, small movements which often result in bad image quality, especially for specialized sequences such as diffusion weighted imaging. For quantitative imaging, such as diffusion, the detected patient motion can be used to improve the quantification of the signal.

Fig. 2 illustrates the pressure sensing system 100 being used to sense pressure exerted by a patient, as performed for example during diagnostic X-ray imaging.

For correct diagnostic medical imaging, e.g. diagnostic X-ray imaging (DXR), images of the part of the body that needs inspection should be positioned in the right way for optimal detection of problems. In a typical imaging scenario, the technologist puts the patient in the required pose, which sometimes can be a painful one, walks back to the control room to presses the scan button. The scanning can take only a fraction of a second, but the time between posing and recording can take several seconds. In addition, when multiple images are taken that are stitched together in post-processing (for example when imaging the spine), there is a delay between the taking of the consecutive images. It may not always be clear to the patient that a subsequent image will be taken and that they should maintain the pose after the previous image was taken. In the time that the technologist walks to the control room after positioning the patient, patients often move slightly, because the pose was forced or painful. From the control room, the technologist cannot always observe small modification of the pose, which can have a large impact on the diagnostic quality of the image. This makes a retake necessary and complicates the workflow. According to the present disclosure, the pressure sensing system 100 may therefore be used automatically to detect changes in position between positioning and image acquisition and to signal these, for example to the technologist and/or to the patient, for example via an appropriate user interface, such as that 810, 812 of the patient state monitoring system 800.

In nearly all poses, the patient exerts some pressure which can be detected by the pressure sensing system 100. For example, the patient may exert pressure on the X-ray detector, which can be detected by the pressure sensing system 100 mounted on the detector. As soon as the technologist has installed the right pose, they can indicate this, as described further below. From that moment, the pressure sensing system 100 may be used to detect pressure changes and to signal to the technologist and/or to the patient if any such changes occur. Before taking the image, the technologist can inspect the detected pressure changes and decide on continuation or repositioning. The pressure sensing system 100 may thus advantageously be used to measure and act upon such pose changes without using 2D or 3D cameras, which can also detect pose changes but which lack sensitivity and which may also pose privacy issues to both patients and technologists.

Various means of user interfacing may be used to exchange information between users and the pressure sensing system 100. For example, a 'freeze' button may be provided as an integral or attachable component of the pressure sensing system 100 for use by the technologist to indicate that the patient is in the correct pose and that the current output of the pressure sensing system 100 should be captured as a reference. Additionally or alternatively, the user interface 810, 812 of the patient state monitoring system 800 may be used to implement the 'freeze' button (and/or to signal the current situation in comparison to the reference situation to one or both of the technologist and the patient). Additionally or alternatively, voice commands and/or interaction with the pressure sensing system 100 itself (e.g. via tap gestures, such as a quick double tap in the right upper corner) may be used to trigger capture of the reference situation.

When the patient state monitoring system 800 receives such a signal indicating that the current pressure should be captured as a reference, the current pressure may be stored as a reference pressure footprint. At least one algorithm executed by the patient state monitoring system 800 may continuously compare the current pressure received from the pressure sensing system 100 to this reference pressure footprint to detect relevant changes. A user interface, such as that of the patient state monitoring system 800, may then signal the current change in pressure compared to the pressure footprint, to the technologist and/or to the patient. The indicated change can take a variety of forms and shapes, from a simple traffic light indicating the size of change to a detailed true and reference pose. The determination as to whether a pressure change is significant (e.g., for the traffic light visualization) may be based on maximum allowed amounts of pressure change per pose. To this end, the pose may be known to the system (e.g., following indication by the technologist on-site or from an Electronic Patient Dossier (EPD), which can state which kind of exam is needed).

In addition to, or instead of, signaling (significant) changes to the patient, the user interface may be used to help the patient to reposition back to the reference pressure footprint. For rigid body parts (like the head or foot) the pressure sensing system 100 can estimate the amount of motion (e.g., 3 degrees of freedom for translation and 3 degrees of freedom for rotation) and can show the patient the start position and the current situation, optionally together with an indication of the corrective action needed. Alternatively, an more abstract view such as a bulls eye can be used to indicate the target position.

Additionally or alternatively, the patient state monitoring system 800 may know when image acquisition is finished and signal to the patient that the scan is done, i.e., that the pose does not need to be maintained any longer, which can provide a celebrative moment for the patient. The patient state monitoring system 800 can also use this information to asses the patient's performance in terms of the amount of movement between the reference pressure footprint and the pressure footprint sensed during scanning. This information can be presented by the UI for the patient and/or technologist. Additionally or alternatively, the information may be used as input for automatic correction of images (e.g., to facilitate stitching).

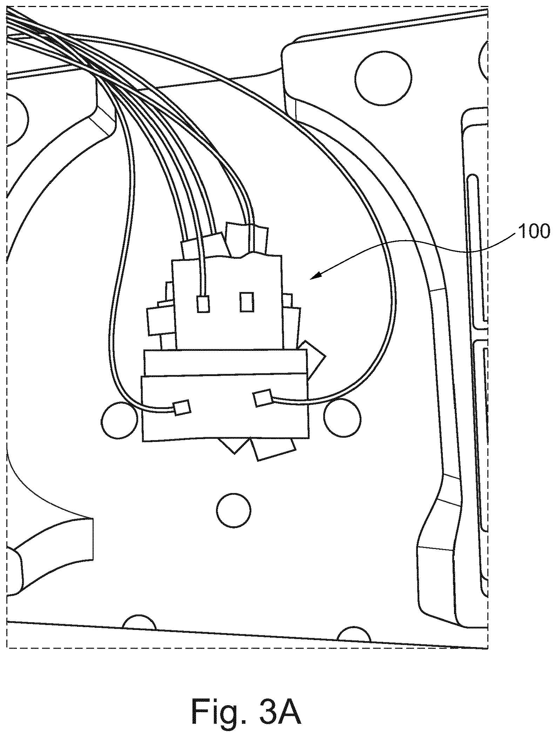

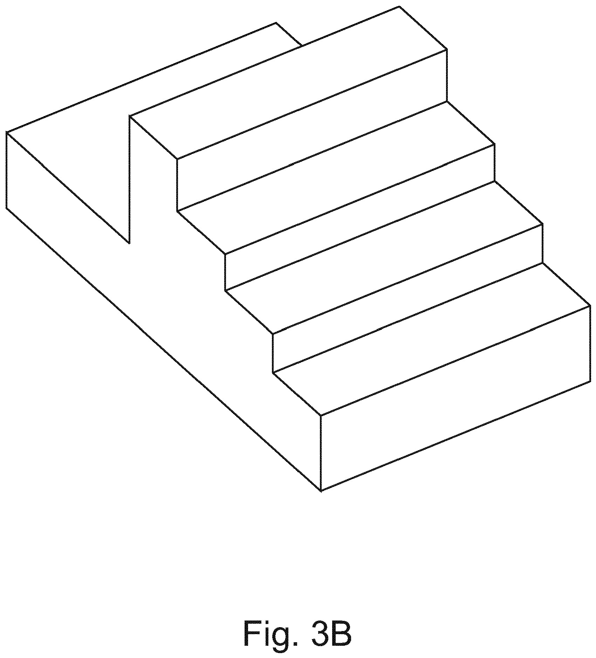

Besides pressure from the patient themselves, pose assistance material may be used to support the patient in a certain pose. Figs. 3A and 3B illustrate such pose assistance material, which may be used to improve the pressure footprint to better detect pose changes. Knowledge of the pose assistance material may thus be used in the algorithm executed by the patient state monitoring system 800 to improve the detection of (significant) changes. For example, if the foot needs to be imaged in a 45 degree angle relative to the horizontal, only the heel is available to exert pressure on the pressure sensing system 100. If the foot is rested on a piece of foam, such as that illustrated in Fig. 3B, the pressure footprint becomes larger such that pressure changes become easier to detect.

Furthermore, the reference pressure footprint may be stored in the patients record for future use. When the patient needs a follow-up scan, the technologist can use the stored reference pressure footprint to position the patient in the same way, thereby facilitating comparison between the images acquired during that scan with the images from the previous scan. The UI may in this case help the technologist with the positioning (similar to the UI that can be used to indicate to the patient how to reposition themselves upon pressure change detection).

### Use case #2: detection of breathing and beating heart

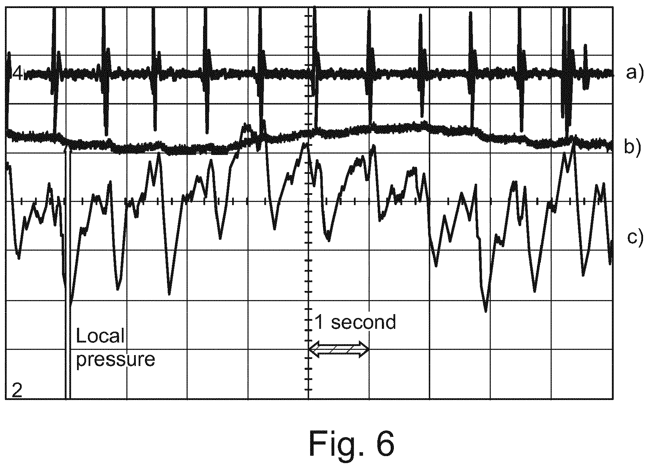

The pressure sensing system 100 may be located between the mattrass and the examination table to measure respiration and heart beating simultaneously, so as to produce signal output as shown in Fig. 6, in which plot a) represents Electro Cardio Gram (ECG) output, plot b) represents output of one of the pressure sensors, which shows the pressure changed caused by breathing, and plot c) represents the bandpass filtered (0.4 to 4 Hz) signal shown in plot b). Breathing and cardio signals are readily separable in the frequency domain: breathing signals have most of their energy around 0.01 to 0.4 Hz and cardio signals around 0.4 to 4 Hz. The ballistic heart pulse detected in this way can be used for cardio timing of MR scans.

### Use case #3: detecting patient body parts

The patient state monitoring system 800 may be configured to estimate the position of parts of the patient such as the organs. Since the pressure sensing system 100 is able to sense the whole body, the patient state monitoring system 800 is able to determine whether the patient lies `feet first' or 'head first' and can also estimate the weight and height of the patient. Based on this information, the system 800 can estimate where for example the liver is located. Thus, in the case that an MR machine is directed to examine a specific organ, the position of that organ as estimated by the patient state monitoring system 800 may be used to position the organ in the image center of the bore. This saves time for the technologist to (re)position the patient in the bore.

Moreover, the patient state monitoring system 800 may be configured to detect changes in body measurements, such as weight or height, caused by for instance cancer treatment or by inflammation. Moreover, using the signals output by the pressure sensing system 100, the patient state monitoring system 800 may be configured to detect the filling of organs such as the bladder and/or rectum, as represented by pressure signals slowly ramping up or down.

Any such changes may lead to a region of interest, e.g., a tumor, drifting out of the image. In response to detecting such changes, the patient state monitoring system 800 may suggest a new registration, by prompting the operator to replan the imaging, or the system 800 may automatically instruct the imaging equipment to perform a new registration and optionally also a new scaling.

### Use case #4: alignment of body position and breathing during radiotherapy

For effective radiotherapy (RT), several daily/weekly sessions are needed. During each session, the patient should ideally lie down in exactly the same position for correct radiation location and direction of the target relative to the radiotherapy radiation source. By installing pressure sensing systems 100 on the examination tables of the CT scanner, the MRI scanner, the Xray scanner, and the RT table, and by using the patient state monitoring system 800 to produce visual representation of the previous and current body positions of the patient, the body position and respiration signals of the patient can be harmonized across these distinct systems. For example, a breathing coach can show a 2D combination of previous and current breathing patterns to align different aspects of respiration (such as respiration type, depth and rate), thereby enabling enhanced registration of images acquired using different modalities. In other non-limiting examples, patient motion may be detected and compared between different/consecutive scans/therapy sessions, for example in the case of a previous scan and a new scan, a scout scan and the main scan, a plan scan and a radiotherapeutic procedure, or two consecutive radiotherapeutic sessions.

### Use case #5: breathing characterization

The pressure sensing system 100 can detect respiration at multiple locations in the chest and/or abdomen of the patient, on the basis of which the patient state monitoring system 800 may be configured to estimate the moving trajectory of the target and organs at risk, during respiration, for accurate radiation. A 4D MR session may be used for calibration.

Use case #6: monitor slow changing digestion processes like bladder or rectum filling during imaging or RT

On the basis of signals output by the pressure sensing system 100, the patient state monitoring system 800 can detect the filling of the bladder and/or rectum before or during imaging or RT to estimate the movement impact on target or organs at risk. Such filling may manifest itself as a gradual pressure shift detected in the region of the bladder and/or rectum. In addition, the patient state monitoring system 800 can detect motion in the bowel, caused for example by pockets of air moving through the bowel. Such changes may result in a different region than expected being treated or imaged. In response to detecting such changes, an alert can be given to notify an operator, the radiation plan may be adapted such that the dose is delivered at a slightly different location, the bowel is at least partially excluded, or the treatment may be temporarily stopped when an air pocket moves into the radiation field. Information concerning the change can be used to infer a treatment dose delivery after the scan or perhaps to adjust the margins of the planning target volume in real-time during the treatment (e.g., starting with small margins and increasing them based on the pressure shift).

### Use case #7: collect patient responses during imaging

The pressure sensing system 100 can be used to collect overt patient responses. By selection (and calibration) of sensors near the hand, the pressure sensing system 100 can function as a button-box (to detect tap gestures) or a trackpad (to detect swipe gestures when moving a finger over the pressure sensing system 100). The pressure sensing system 100 can thus replace the patient alarm balloon, MRI-compatible button-boxes, trackpad and other peripheral input devices. This facilitates task-based fMRI, real-time neurofeedback, and any other type of imaging that requires patient input without the need for other peripheral devices.

### Use case #8: motion characterization

The 2D design of the pressure sensing system 100 allows patient motion to be detected in more than one direction, which is generally done for so-called "motion surrogates". Examples of "motion surrogates" include a breathing belt around the torso (to measure the expanding torso during breathing) and a short MRI navigator scan which seeks to measure the position of the diaphragm. This can be used to detect changes in respiration, e.g. from chest to belly breathing to back breathing or vice versa. Additionally, it can be used to perform multi-dimensional sorting of 4D-MRI data (i.e., "binning"). Current 4D-MRI implementations generally sort the data based on a 1D signal (provided for example by bellows or an internal MRI surrogate). By performing the sorting process in multiple directions, a more representative 4D-MRI can be generated, and the variation in breathing can be better characterized.

### Use case #9: consistent change in motion to trigger intrafractional plan update

Detecting patient motion during treatment in the manner described herein may be used to trigger a temporary stop in irradiation, a complete stop, or a plan update to improve the treatment during every fraction. Different motion, such as coughing, swallowing, bulk motion or breathing can have different effects on the treatment and some are less problematic than others. The motion event can result in a different position after the motion than before. This change is more problematic than the short motion event, since, for the remainder of the treatment, a different anatomy will be irradiated than expected. The patient state monitoring system 800 may therefore be configured to detect the position of the patient after the motion event, to compare the detected position with the initial position, and to issue a warning when the detected position is significantly different from the initial position. The patient state monitoring system 800 may be further configured to provide feedback and/or guidance to the patient and/or staff on how to correct the patient position.

### Use case #10: continuous motion assessment for MR-guided RT

For MRI-guided treatments, for example on the MR-Linac, a daily anatomical scan is made at the beginning of each daily fraction. This scan is registered to the planning scan to check if the RT plan is still acceptable. If this is not the case, delineations of the tumor and nearby healthy tissue are updated, and a new plan is generated. Generating a new plan takes anywhere between several seconds to several minutes, depending on how much work has to be done. After a new plan is made, another anatomical scan has to be made (as in the beginning) to check that the patient is still positioned in the same way as in the beginning (on which the new plan is based). This is called the position verification scan. The pressure sensing system 100 may be used to generate a fast surrogate signal that can be acquired continuously while the new plan is generated. Advantageously, this motion signal does not interfere with MR imaging, since other scans may be acquired during this `idle' time, such as a quantitative image. Since the motion of the patient is continuously monitored, an additional position verification scan is not needed, leading to a shorter time on the treatment table, and the replanning process can be stopped if the patient moves. If the surrogate is quantitative, the detected motion may furthermore be used to perform a 'virtual couch shift' to eliminate the need for replanning, as described hereinabove.

### Use case #11: safety detection of skin-to-skin contact

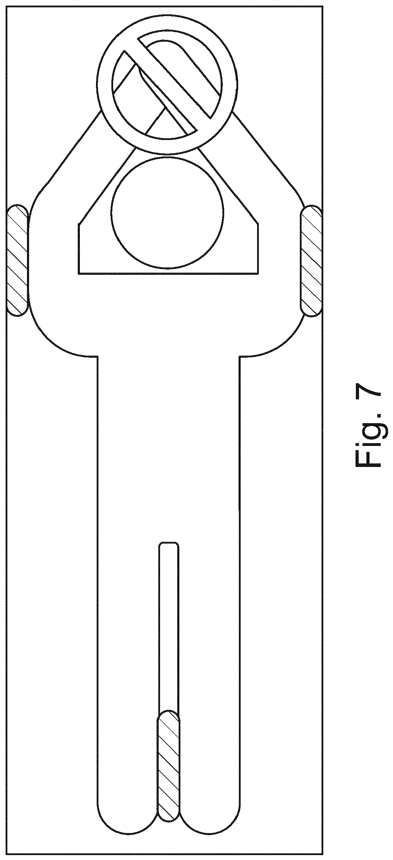

Skin-to-skin contact (even with a small gap in between areas of skin) by the patient during an MRI scan can lead to serious skin burns due to heating of the skin, as illustrated in Fig. 7. This is likely caused when the changing magnetic fields in the MRI scanner lead to electric current flowing in the "closed" skin loops. The pressure sensing system 100 can be used to detect this, whereupon the patient state monitoring system 800 may be configured to issue a warning, for example to the operator and potentially also to the patient, so that corrective action can be taken. To that end, the pressure sensing system 100 may be shaped and sized as a full body pressure sensor. Burns may also result from direct contact between the patient and transmitting radiofrequency (RF) coils or other parts of the MR system, such as heated loops in external cables touching the patient. The pressure sensing system 100 may also be used to detect this.

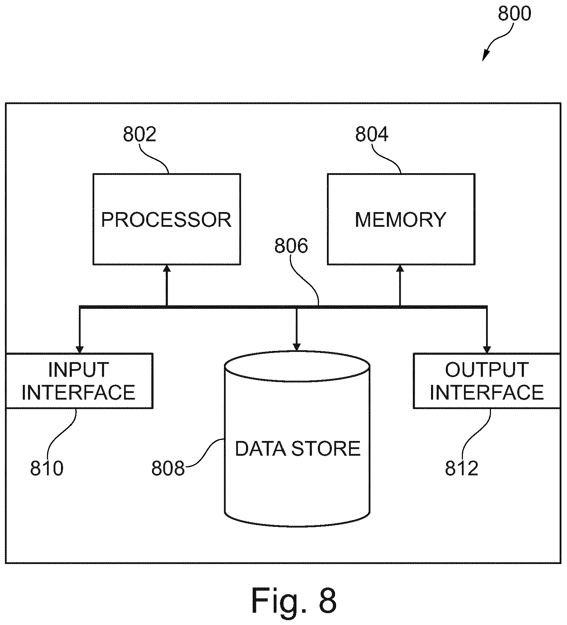

Fig. 8 illustrates an exemplary computing system 800 that can be used in accordance with the systems and methods disclosed herein. In particular, the computing system may be used to implement the patient state monitoring system 800 described herein. The computing system 800 may form part of or comprise any desktop, laptop, server, or cloud-based computing system. The computing system 800 includes at least one processor 802 that executes instructions that are stored in a memory 804. The instructions may be, for instance, instructions for implementing functionality described as being carried out by one or more components described herein or instructions for implementing one or more of the methods described herein. The processor 802 may access the memory 804 by way of a system bus 806. In addition to storing executable instructions, the memory 804 may also store conversational inputs, scores assigned to the conversational inputs, etc.

The computing system 800 additionally includes a data store 808 that is accessible by the processor 802 by way of the system bus 806. The data store 808 may include executable instructions, log data, etc. The computing system 800 also includes an input interface 810 that allows external devices to communicate with the computing system 800. For instance, the input interface 810 may be used to receive instructions from an external computer device, from a user, etc. The computing system 800 also includes an output interface 812 that interfaces the computing system 800 with one or more external devices. For example, the computing system 800 may display text, images, etc. by way of the output interface 812.

It is contemplated that the external devices that communicate with the computing system 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing system 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing system 800 may be a distributed system. Thus, for instance, several devices may be in communication by way of a network connection and may collectively perform tasks described as being performed by the computing system 800.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. A method for monitoring patient state in connection with a medical procedure, the method comprising:
obtaining pressure signals from at least one optical waveguide pressure sensor array arranged to interact with the patient; and
processing the pressure signals to monitor patient state.

2. The method of claim 1, wherein processing the pressure signals to monitor patient state comprises determining a location of one or more parts of the patient.

3. The method of claim 2, further comprising using the determined location of the one or more parts of the patient in patient positioning.

4. The method of any preceding claim, wherein the medical procedure comprises an image acquisition process, the method further comprising using the monitored patient state for protocol setting in relation to the image acquisition process.

5. The method of any preceding claim, further comprising using the pressure signals obtained from at least one optical waveguide pressure sensor array for placement of equipment in connection with the medical procedure.

6. The method of any preceding claim, wherein processing the pressure signals comprises detecting patient motion, and estimating a magnitude of the detected patient motion.

7. The method of any preceding claim, wherein processing the pressure signals comprises detecting patient motion, the method further comprising, in response to detecting the patient motion, outputting one or more output signals for triggering remedial action.

8. The method of claim 7, wherein the medical procedure comprises an image acquisition process, and wherein the remedial action comprises adapting the image acquisition process in response to detecting the patient motion.

9. The method of claim 7 or 8, wherein the remedial action comprise notifying one or more users of the detected patient motion.

10. The method of any preceding claim, wherein processing the pressure signals comprises detecting patient motion, wherein detecting patient motion comprises detecting patient respiratory activity, the method further comprising using the detected patient respiratory activity to characterize patient breathing.

11. The method of any preceding claim, wherein processing the pressure signals comprises detecting patient motion, and wherein detecting patient motion comprises detecting patient cardiac activity.

12. The method of any preceding claim, wherein processing the pressure signals to monitor patient state comprises recognizing one or more gestures performed by the patient to provide user input.

13. The method of any preceding claim, wherein the medical procedure comprises an image acquisition process, the method further comprising using data related to a detected change in patient position as input for one or more subsequent image processing steps.

14. The method of any preceding claim, wherein the medical procedure comprises an image acquisition process, and wherein the image acquisition process comprises quantitative imaging, the method further comprising using the detected patient motion to improve signal quantification.

15. The method of any preceding claim, further comprising obtaining further pressure signals from the at least one optical waveguide pressure sensor array during a planning phase prior to the medical procedure, and verifying that the patient position remains unchanged during the planning phase.

16. The method of any preceding claim, wherein processing the pressure signals to monitor patient state comprises detecting contact or proximity during image acquisition process between one or more parts of the patient and one or more dangerous objects.

17. A patient state monitoring system configured to perform the method of any preceding claim.
